(19) Europäisches Patentamt
European Patent Office
Office européen des brevets

(11) **EP 4 090 308 B1**

(12) **EUROPEAN PATENT SPECIFICATION**

(45) Date of publication and mention
of the grant of the patent:
**26.07.2023 Bulletin 2023/30**

(21) Application number: **21701053.7**

(22) Date of filing: **12.01.2021**

(51) International Patent Classification (IPC):
*A61K 8/31* (2006.01)   *A61K 8/34* (2006.01)
*A61K 8/41* (2006.01)   *A61K 8/49* (2006.01)
*A61K 8/891* (2006.01)   *A61K 8/892* (2006.01)
*A61Q 5/00* (2006.01)

(52) Cooperative Patent Classification (CPC):
**A61K 8/31; A61K 8/34; A61K 8/41; A61K 8/49;
A61K 8/891; A61K 8/892; A61Q 5/00**

(86) International application number:
**PCT/EP2021/050417**

(87) International publication number:
**WO 2021/144231 (22.07.2021 Gazette 2021/29)**

(54) **HAIR CARE COMPOSITION**

HAARPFLEGEZUSAMMENSETZUNG

COMPOSITION DE SOINS CAPILLAIRES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **15.01.2020 PCT/CN2020/072299
18.02.2020 EP 20157979**

(43) Date of publication of application:
**23.11.2022 Bulletin 2022/47**

(73) Proprietors:
• **Unilever IP Holdings B.V.**
**3013 AL Rotterdam (NL)**
Designated Contracting States:
**AL AT BE BG CH CZ DK EE ES FI FR GR HR HU
IS LI LT LU LV MC MK NL NO PL PT RO SE SI SK
SM**
• **UNILEVER GLOBAL IP LIMITED**
**Wirral**
**Merseyside CH62 4ZD (GB)**
Designated Contracting States:
**CY DE GB IE IT MT RS TR**

(72) Inventors:
• **CAO, Qunhua**
**Shanghai, 200335 (CN)**
• **QI, Lucheng**
**Shanghai, 200335 (CN)**

(74) Representative: **Mathai, Neenu Grace**
**Unilever Patent Group**
**Bronland 14**
**6708 WH Wageningen (NL)**

(56) References cited:
**WO-A1-2017/133874   WO-A1-2018/145941**

• **"USE OF DIMETHICONOL AND DIMETHICONE
GUM BLENDS IN PERSONAL CARE
APPLICATIONS", IP.COM, IP.COM INC., WEST
HENRIETTA, NY, US, 16 April 2019 (2019-04-16),
XP013182953, ISSN: 1533-0001**
• **Judy Zhu ET AL: "The Beauty of Silicone in Hair
Care Applications", , 1 December 2017
(2017-12-01), XP055715051, Retrieved from the
Internet:
URL:https://www.dow.com/documents/en-us/te
ch-art/27/27-15/27-1550-01-the-beauty-of-s
ilicone-in-hair-care-application.pdf?ifram e=true
[retrieved on 2020-07-15]**

**Description**

**Technical Field of the Invention**

This invention relates to hair care compositions, more particularly to hair care compositions comprising specific alkanes, anti-dandruff agents and silicone compounds.

**Background of the Invention**

Hair care compositions generally provide cleansing or conditioning benefits or a combination of the two. Hair shampoos typically comprise one or more cleansing surfactants which generally aid in cleaning the hair and the scalp free of undesirable soil, particles and fatty matter. Hair conditioners are another class of hair care composition which are generally used on hair in the wet condition after the hair has been washed with a shampoo. Compositions which provide the dual benefits of shampooing and conditioning are also known.

Hair conditioners generally comprise cationic surfactants. The conditioning benefit is achieved by including one or more conditioning agents in the hair care composition. Typically, the most popular conditioning agents used in hair care compositions are waterinsoluble oily materials such as mineral oils, naturally occurring oils such as triglycerides and silicone compounds. Conditioning benefit is achieved by the oily material being deposited onto the hair resulting in the formation of a film, which makes the hair easier to comb when wet and more manageable when dry. The most popular hair conditioning agents used are silicone compounds.

WO 2017/133874 discloses a composition for hair comprising (a) 0.1 to 10% by weight of a silicone compound; (b) 0.1 to 5% by weight of a cationic surfactant; (c) 0.01 to 5% by weight of a zinc antidandruff agent; (d) 0 to 1% of a cyclomethicone compound; and (e) a stabilizing polymer selected from poloxamers. The disclosed compositions do not comprise a linear or branched alkane having from 8 to 22 carbon atoms and anti-dandruff agents as defined in part (d) of claim 1.

However, preferred hair care composition will provide more benefits than simple cleansing and conditioning. For example, it is desirable to incorporate various benefit agents in hair care composition to provide benefits in addition to cleansing and conditioning. For example, anti-dandruff benefit has been provided through hair care compositions. Dandruff is an issue that affects many people globally. The condition is manifested by the shedding of clumps of dead skin cells from the scalp. These are white in colour and provide an aesthetically displeasing appearance. A factor that contributes to dandruff is certain members of the Malassezia yeasts. To combat these, anti-dandruff products have included certain anti-dandruff agents which have anti-fungal activity, for example, piroctone olamine (Octopirox®), zinc pyrithione, azole based anti-fungal agents (e.g. climbazole, ketoconazole), selenium sulfide or combinations thereof.

The present invention relates to a hair conditioning composition comprising a combination of a cationic surfactant and a silicone compound. When an anti-dandruff agent is included in a hair conditioning composition of this type, there is the so-called silicone fouling problem where the stability of the composition is compromised with the formation of soft grits which can foul process machinery and produce a product with lower stability. The present inventors have now found unexpectedly that specific alkanes can be used to minimize this silicone fouling problem and enhance the stability of such hair compositions.

**Summary of the Invention**

In a first aspect, the present invention is directed to a hair care composition comprising:

(a) a cationic surfactant;
(b) a silicone compound;
(c) a linear or branched alkane having from 8 to 22 carbon atoms; and
(d) an anti-dandruff agent selected from piroctone olamine, selenium sulfide, azole based anti-fungal agents and mixtures thereof;

   wherein the composition is substantially free of a cyclomethicone compound;
   wherein said cyclomethicone compound is present in an amount less than 1.5% based on total weight of the hair care composition.

All other aspects of the present invention will more readily become apparent upon considering the detailed description and examples which follow.

## Detailed Description

Except in the examples, or where otherwise explicitly indicated, all numbers in this description indicating amounts of material or conditions of reaction, physical properties of materials and/or use may optionally be understood as modified by the word "about".

All amounts are by weight of the final hair care composition, unless otherwise specified. It should be noted that in specifying any ranges of values, any particular upper value can be associated with any particular lower value.

"Hair care composition", as used herein, is meant to include a composition for topical application to hair and/or scalp of mammals, especially humans. Such a composition may be generally classified as leave-on or rinse off, and includes any product applied to a human body for also improving appearance, cleansing, odor control or general aesthetics. The composition of the present invention can be in the form of a liquid, lotion, cream, foam, scrub, gel, or bar. Non-limiting examples of such compositions include leave-on hair lotions, creams, and rinse-off shampoos, conditioners, shower gels, or toilet bar. The composition of the present invention is preferably a rinse-off composition, especially preferred being a shampoo or a conditioner and most preferably a conditioner.

The hair care composition comprises conditioning surfactants selected from cationic surfactants, used singly or in admixture. Preferably, the cationic surfactants have the formula $N^+R^1R^2R^3R^4$ wherein $R^1$, $R^2$, $R^3$ and $R^4$ are independently ($C_1$ to $C_{30}$) alkyl or benzyl. Preferably, one, two or three of $R^1$, $R^2$, $R^3$ and $R^4$ are independently ($C_4$ to $C_{30}$) alkyl and the other $R^1$, $R^2$, $R^3$ and $R^4$ group or groups are ($C_1$-$C_6$) alkyl or benzyl. More preferably, one or two of $R^1$, $R^2$, $R^3$ and $R^4$ are independently ($C_6$ to $C_{30}$) alkyl and the other $R^1$, $R^2$, $R^3$ and $R^4$ groups are ($C_1$-$C_6$) alkyl or benzyl groups. Optionally, the alkyl groups may comprise one or more ester (-OCO- or -COO-) and/or ether (-O-) linkages within the alkyl chain. Alkyl groups may optionally be substituted with one or more hydroxyl groups. Alkyl groups may be straight chain or branched and, for alkyl groups having 3 or more carbon atoms, cyclic. The alkyl groups may be saturated or may contain one or more carbon-carbon double bonds (eg, oleyl). Alkyl groups are optionally ethoxylated on the alkyl chain with one or more ethyleneoxy groups.

Suitable cationic surfactants for use in hair care compositions of the present invention include cetyltrimethylammonium chloride, behentrimethylammonium chloride, cetylpyridinium chloride, tetramethylammonium chloride, tetraethylammonium chloride, octyltrimethylammonium chloride, dodecyltrimethylammonium chloride, hexadecyltrimethylammonium chloride, octyldimethylbenzylammonium chloride, decyldimethylbenzylammonium chloride, stearyldimethylbenzylammonium chloride, didodecyldimethylammonium chloride, dioctadecyldimethylammonium chloride, tallowtrimethylammonium chloride, dihydrogenated tallow dimethyl ammonium chloride (eg, Arquad 2HT/75 from Akzo Nobel), cocotrimethylammonium chloride, PEG-2-oleammonium chloride and the corresponding hydroxides thereof. Further suitable cationic surfactants include those materials having the CTFA designations Quaternium-5, Quaternium-31 and Quaternium-18. Mixtures of any of the foregoing materials may also be suitable. A particularly useful cationic surfactant for use in conditioners according to the invention is cetyltrimethylammonium chloride, available commercially, for example as GENAMIN CTAC, ex Hoechst Celanese. Another particularly useful cationic surfactant for use in conditioners according to the invention is behenyltrimethylammonium chloride, available commercially, for example as GENAMIN KDMP, ex Clariant. Yet another preferred cationic surfactant is stearamidopropyl dimethylamine. The most preferred cationic surfactants for use in the compositions of present invention are stearamidopropyl dimethylamine, behentrimonium chloride, cetyltrimethylammonium chloride or mixtures thereof.

The hair care composition typically comprises the cationic surfactant in an amount of from 0.1 to 5%, more preferably from 0.5 to 2.5%, based on total weight of the hair care composition and including all ranges subsumed therein.

The hair care composition of the present invention comprises a silicone compound which is not a cyclomethicone. Preferably, the silicone compound is a non-volatile silicone. "Non-volatile", as used herein, means that the silicone has a vapor pressure less than 1000 Pa at 25°C and one atmospheric pressure.

Preferably, the silicone compound is emulsified droplets of silicone oil, which has a mean droplet diameter ($D_{3,2}$) of less than 15 $\mu$m, preferably less than 10 $\mu$m, more preferably less than 5 $\mu$m. Preferably the mean droplet diameter ($D_{3,2}$) of a silicone oil is greater than 0.05 $\mu$m, more preferably greater than 0.1 $\mu$m, ideally from 0.1 $\mu$m to 5 $\mu$m. The mean droplet diameter ($D_{3,2}$) of a silicone oil may be measured by means of a laser light scattering technique, for example using a 2600D Particle Sizer from Malvern Instruments.

Suitable silicone compounds include polysiloxanes, in particular polydimethylsiloxanes which have the CTFA designation dimethicone. Also suitable for use in compositions of this invention (particularly shampoos and conditioners) are polydimethylsiloxanes having hydroxyl end groups, which have the CTFA designation dimethiconol. Another class of silicones which may be used are functionalized silicones such as amino functional silicones, meaning a silicone containing at least one primary, secondary or tertiary amine group, or a quaternary ammonium group. Examples of suitable amino functional silicones include polysiloxanes having the CTFA designation "amodimethicone." Also suitable for use in compositions of this invention are silicone gums having a slight degree of crosslinking, as are described for example in WO 96/31188. Preferably, the silicone compound comprises dimethicone, dimethiconol, amodimethicone, derivatives or mixtures thereof.

In another preferred embodiment, the silicone compound is dimethiconol derivatives including dimethiconol/silsesquioxane copolymer, dimethiconol arginine, dimethiconol beeswax, dimethiconol behenate, dimethiconol cysteine, dimethiconol meadowfoamate, dimethiconol methionine, dimethiconol panthenol, dimethiconol stearate, trimethylsiloxysilicate/dimethiconol crosspolymer, acrylates/dimethiconol acrylate copolymer or mixtures thereof. Particularly preferred is dimethiconol/silsesquioxane copolymer.

The viscosity of a preferred silicone compound used as an emulsified silicone oil itself is typically at least 10,000 cSt (centi-Stokes=$mm^2 \cdot S^{-1}$) at 25°C, preferably at least 60,000 cSt, most preferably at least 500,000 cSt, ideally at least 1,000,000 cSt. Preferably the viscosity does not exceed $10^9$ cSt for ease of formulation. Viscosity of silicone can be determined, for example, by the relevant international standard, such as ISO 3104.

Suitable emulsified silicones for use in the hair care compositions of this invention are available as pre-formed silicone emulsions from suppliers of silicones such as Dow Silicones Corporation and GE silicones. The use of such pre-formed silicone emulsion is preferred for ease of processing and control of silicone particle size. Such pre-formed silicone emulsions will typically additionally comprise a suitable emulsifier, and may be prepared by a chemical emulsification process such as emulsion polymerisation, or by mechanical emulsification using a high shear mixer. Examples of suitable pre-formed silicone emulsions include Dowsil 1785 BA, MEM 7134 POE / MEM7154, Dowsil CE-7126 all available from Dow. These are emulsions of dimethiconol/dimethicone, amodimethicone. Another example of suitable pre-formed silicone emulsion includes dimethiconol/silsesquioxane copolymer under the trade name CE-1607 IHD emulsion from Dow.

The silicone compound is generally present in hair care composition of this invention in an amount from 0.1 to 10%, more preferably from 0.5 to 5%, most preferably from 0.5 to 3%, based on total weight of the hair care composition and including all ranges subsumed therein.

The hair care composition further comprises a linear or branched alkane having from 8 to 22 carbon atoms, preferably from 10 to 20, more preferably from 12 to 18. Illustrative examples of the alkanes that may be used in this invention include, for example, octane, isooctane, decane, isodecane, dodecane, isododecane, tetradecane, isotetradecane, hexadecane, isohexadecane, octodecane, isooctadecane, eicosane, isoeicosane, docosane, isodocosane or mixtures thereof. Preferably, the alkane is branched alkane comprising isooctane, isodecane, isododecane, isotetradecane, isohexadecane, isooctadecane, isoeicosane, isodocosane or mixtures thereof. Isohexadecane is particularly preferred.

The linear or branched alkane is preferably present in hair care composition of the present invention at a level ranging from 0.01 to 5%, more preferably from 0.05 to 3%, most preferably from 0.1 to 2%, based on total weight of the hair care composition and including all ranges subsumed therein.

The anti-dandruff agents present in the hair care composition are compounds that are active against dandruff and are typically anti-microbial agents and preferably anti-fungal agents. Suitable anti-dandruff agents that may be used in this invention are selected from piroctone olamine (Octopirox®), selenium sulfide and mixtures thereof. In a preferred embodiment, the antidandruff agent is piroctone olamine.

Typically, the hair care composition of the invention comprises the anti-dandruff agent in an amount of from 0.01 to 10%, more preferably from 0.01 to 5%, more preferably still from 0.05 to 2%, and most preferably from 0.05 to 1.5%, based on total weight of the hair care composition and including all ranges subsumed therein.

The pH of the composition is preferably equal to or higher than 4.0, more preferably in the range of 4.0 to 7.0.

The hair care composition of the present invention is substantially free of a cyclomethicone compound. "Substantially free of", as used herein, means that a cyclomethicone compound is present in an amount less than 1.5%, preferably less than 1.0%, more preferably less than 0.75%, more preferably still less than 0.5%, and even more preferably less than 0.1% and most preferably absent in the composition, based on total weight of the hair care composition and including all ranges subsumed therein. Examples of suitable cyclomethicone compounds include octamethylcyclotetrasiloxane, decamethylcyclopentasiloxane, dodecamethylcyclohexasiloxane or mixtures thereof. The most commonly used cyclomethicone compound is decamethylcyclopentasiloxane which is known as D5 and it is preferred that the hair care composition of the present invention is substantially free of D5.

Hair care composition of the present invention may additionally comprise a fatty alcohol. The combined use of fatty alcohols and cationic surfactants is believed to be especially advantageous, because this leads to the formation of a lamellar phase, in which the cationic surfactant is dispersed.

Representative fatty alcohols comprise from 8 to 22 carbon atoms, more preferably 16 to 22. Fatty alcohols are typically compounds containing straight chain alkyl groups. Examples of suitable fatty alcohols include cetyl alcohol, stearyl alcohol and mixtures thereof. The use of these materials is also advantageous in that they contribute to the overall conditioning properties of compositions of the invention.

The fatty alcohol is preferably present in the hair care composition of the present invention at a level ranging from 0.5 to 10%, more preferably from 0.1 to 8%, more preferably still from 0.2 to 7% and most preferably from 0.3 to 6%, based on total weight of the hair care composition and including all ranges subsumed therein. The weight ratio of cationic surfactant to fatty alcohol is typically from 1:1 to 1:10, more preferably from 1:1.5 to 1:8, most preferably from 1:2 to 1:5.

Preservatives may also be incorporated into the hair care composition of this invention to protect against the growth of potentially harmful microorganisms. Suitable traditional preservatives include alkyl esters of parahydroxybenzoic acid, hydantoin derivatives, propionate salts, and a variety of quaternary ammonium compounds. Illustrative yet non-limiting examples of the types of preservatives that may be used in this invention include, for example, phenoxyethanol, sodium salicylate, methyl paraben, butyl paraben, propyl paraben, diazolidinyl urea, sodium dehydroacetate, 1,2-hexanediol, benzyl alcohol, sodium benzoate, iodopropynyl butylcarbamate, caprylyl glycol, disodium EDTA or mixtures thereof. In an especially preferred embodiment, the preservative is 1,2-hexanediol, benzyl alcohol or a mixture thereof. Preservatives are preferably employed in amounts ranging from 0.01 to 2% by weight of the hair care composition.

The hair care composition of the present invention may contain other ingredients which are common in the art to enhance physical properties and performances. Suitable ingredients include but are not limited to fragrance, dyes and pigments, pH adjusting agents, pearlescers or opacifiers, viscosity modifiers, thickeners, and natural hair nutrients such as botanicals, fruit extracts, sugar derivatives and amino acids.

The compositions of the invention are primarily intended for topical application to scalp and/or at least a portion of the hair of an individual, either in rinse-off or leave-on compositions, preferably in rinse-off compositions like conditioners.

The following examples are provided to facilitate an understanding of the present invention. The examples are not provided to limit the scope of the claims.

## Examples

Example 1

This example demonstrates the reduced silicone fouling by using alkanes. Compositions were prepared according to the formulations detailed in Table 1. All ingredients are expressed by weight percent of the total formulation, and as level of active ingredient.

**Table 1**

| Ingredient | Samples | | | | | | | |
|---|---|---|---|---|---|---|---|---|
| | 1 | 2 | 3 | 4 | 5 | 6 | 7 | 8 |
| Water | To 100 | To 100 | To 100 | To 100 | To 100 | To 100 | To 100 | To 100 |
| Cetearyl alcohol | 4.00 | 4.00 | 4.00 | 4.00 | 4.00 | 4.00 | 4.00 | 4.00 |
| Behentrimonium chloride | 0.70 | 0.70 | 0.70 | 0.70 | 0.70 | 0.70 | 0.70 | 0.70 |
| Stearamidopropyl dimethylamine | 1.00 | 1.00 | 1.00 | 1.00 | 1.00 | 1.00 | 1.00 | 1.00 |
| Lactic acid | 0.361 | 0.361 | 0.361 | 0.361 | 0.361 | 0.361 | 0.361 | 0.361 |
| Sodium chloride | 0.085 | 0.085 | 0.085 | 0.085 | 0.085 | 0.085 | 0.085 | 0.15 |
| Perfume | 0.75 | 0.75 | 0.75 | 0.75 | 0.75 | 0.75 | 0.75 | 0.75 |
| Piroctone olamine | 0.25 | 0.15 | 0.15 | 0.15 | 0.15 | 0.15 | 0.15 | 0.15 |
| Dimethiconol[a] | 2.00 | 2.00 | 2.00 | 2.00 | 2.00 | 2.00 | 2.00 | -- |
| Dimethiconol/silsesquioxane copolymer and isohexadecane[b] | -- | -- | -- | -- | -- | -- | -- | 1.50 |
| n-dodecane | -- | -- | 0.80 | -- | -- | -- | -- | -- |
| Isododecane | -- | -- | -- | 0.80 | -- | -- | -- | -- |
| n-tetradecane | -- | -- | -- | -- | 0.80 | -- | -- | -- |
| n-hexadecane | -- | -- | -- | -- | -- | 0.80 | -- | -- |
| n-octadecane | -- | -- | -- | -- | -- | -- | 0.80 | -- |
| Propylene glycol | 0.30 | 0.30 | 0.30 | 0.30 | 0.30 | 0.30 | 0.30 | 0.30 |

(continued)

| Ingredient | Samples | | | | | | | |
|---|---|---|---|---|---|---|---|---|
| | 1 | 2 | 3 | 4 | 5 | 6 | 7 | 8 |
| 1,2-hexanediol | 0.20 | 0.20 | 0.20 | 0.20 | 0.20 | 0.20 | 0.20 | 0.20 |
| Benzyl alcohol | 0.20 | 0.20 | 0.20 | 0.20 | 0.20 | 0.20 | 0.20 | 0.20 |
| Glycerin | 3.00 | 3.00 | 3.00 | 3.00 | 3.00 | 3.00 | 3.00 | 0.10 |
| a. Commercial dimethiconol under the trade name Dowsil 1785 BA from Dow. b. Commercial material under the trade name CE-1607 IHD emulsion which contains 42 wt% dimethiconol/silsesquioxane copolymer and 28 wt% isohexadecane from Dow. | | | | | | | | |

_Methods_

The %silicone fouling was measured using the following procedure: about 800g of the conditioner composition was taken in a 1000 mL beaker and stirred with a flat paddle at 160 rpm for 20 mins. Then take out 700g conditioner composition and leave 100g in the breaker. Add about 700 g of hot water (80-90°C) into the beaker and stirred with a flat paddle at 160 rpm for 20 mins. The diluted mass was then filtered using a 710 $\mu$m mesh. The contents of the beaker were then filled with 800 mL water (25°C) and stirred at 160 rpm for 1 min. After that, the contents of the beaker were filtered using a 710 $\mu$m mesh. The beaker, the mesh and the paddle were then dried in oven at 45°C for 3 hours. The increase in weight of the beaker, the paddle and the mesh was then measured which was an indicator of the absolute amount of silicone fouling. The % silicone fouling was then calculated using the equation:

$$\% \text{ silicone fouling} = (\text{absolute amount of silicone fouling})/(\text{amount of the hair conditioner composition taken})*100.$$

_Results_

The % silicone fouling are reported in Table 2.

**Table 2**

| | Samples | | | | | | | |
|---|---|---|---|---|---|---|---|---|
| | 1 | 2 | 3 | 4 | 5 | 6 | 7 | 8 |
| % silicone fouling | 12.75 | 3.387 | 0.298 | 0.187 | 0.710 | 0.932 | 0.637 | 0.180 |

Silicone fouling higher than 3% (as per the procedure listed above) is taken as unacceptable. The results clearly showed that samples 3 to 8 comprising alkanes exhibited lower silicone fouling compared to sample 1 and 2.

**Claims**

1.   A hair care composition comprising:

   (a) a cationic surfactant;
   (b) a silicone compound;
   (c) a linear or branched alkane having from 8 to 22 carbon atoms; and
   (d) an anti-dandruff agent selected from piroctone olamine, selenium sulfide, azole based anti-fungal agents and mixtures thereof;

   wherein the composition is substantially free of a cyclomethicone compound; wherein said cyclomethicone compound is present in an amount less than 1.5% based on total weight of the hair care composition.

2.   The hair care composition according to claim 1, wherein the silicone compound is a non-volatile silicone which has a vapor pressure less than 1000 Pa at 25°C and one atmospheric pressure.

3. The hair care composition according to claim 1 or claim 2, wherein the silicone compound is polysiloxane comprising dimethicone, dimethiconol, amodimethicone, dimethiconol derivatives or mixtures thereof.

4. The hair care composition according to claim 3, wherein the silicone compound is dimethiconol derivatives including dimethiconol/silsesquioxane copolymer, dimethiconol arginine, dimethiconol beeswax, dimethiconol behenate, dimethiconol cysteine, dimethiconol meadowfoamate, dimethiconol methionine, dimethiconol panthenol, amodimethicone, dimethiconol stearate, trimethylsiloxysilicate/dimethiconol crosspolymer, acrylates/dimethiconol acrylate copolymer or mixtures thereof, preferably dimethiconol/silsesquioxane copolymer.

5. The hair care composition according to any of the preceding claims, wherein the composition comprises the silicone compound in an amount of from 0.1 to 10% by weight of the composition, preferably from 0.5 to 5%.

6. The hair care composition according to any of the preceding claims, wherein the linear or branched alkane comprises octane, isooctane, decane, isodecane, dodecane, isododecane, tetradecane, isotetradecane, hexadecane, isohexadecane, octodecane, isooctadecane, eicosane, isoeicosane, docosane, isodocosane or mixtures thereof.

7. The hair care composition according to claim 6, wherein the linear or branched alkane comprises isooctane, isodecane, isododecane, isotetradecane, isohexadecane, isooctadecane, isoeicosane, isodocosane or mixtures thereof, preferably isohexadecane.

8. The hair care composition according to any of the preceding claims, wherein the composition comprises the linear or branched alkane in an amount of from 0.01 to 5% by weight of the composition, preferably from 0.05 to 3%.

9. The hair care composition according to any of the preceding claims, wherein the anti-dandruff agent is piroctone olamine.

10. The hair care composition according to any of the preceding claims, wherein the composition comprises the anti-dandruff agent in an amount of from 0.01 to 10% by weight of the composition, preferably from 0.01 to 5%.

11. The hair care composition according to any of the preceding claims, wherein the cyclomethicone compound comprises octamethylcyclotetrasiloxane, decamethylcyclopentasiloxane, dodecamethylcyclohexasiloxane or mixtures thereof, preferably the cyclomethicone compound is decamethylcyclopentasiloxane.

12. The hair care composition according to any of claims 1 to 10, wherein the cyclomethicone compound is absent in the composition.

13. The hair care composition according to any of the preceding claims, wherein the cationic surfactant comprises stearamidopropyl dimethylamine, behentrimonium chloride, cetyltrimethylammonium chloride or mixtures thereof.

14. The hair care composition according to any of the preceding claims, wherein the composition additionally comprises a fatty alcohol.

15. The hair care composition according to claim 14, wherein the fatty alcohol comprises from 8 to 22 carbon atoms.

**Patentansprüche**

1. Haarpflegezusammensetzung, umfassend:

   (a) ein kationisches Tensid;
   (b) eine Silikonverbindung;
   (c) ein lineares oder verzweigtes Alkan mit 8 bis 22 Kohlenstoffatomen; und
   (d) ein Antischuppenmittel, ausgewählt aus Pirocton-Olamin, Selensulfid, Antimykotika auf Azolbasis und Mischungen davon;

   wobei die Zusammensetzung im Wesentlichen frei von einer Cyclomethicon-Verbindung ist, wobei die Cyclomethicon-Verbindung in einer Menge von weniger als 1,5%, bezogen auf das Gesamtgewicht der Haarpflegezusammensetzung, vorliegt.

2. Haarpflegezusammensetzung nach Anspruch 1, wobei die Siliconverbindung ein nichtflüchtiges Silicon ist, das bei 25°C und dem Druck von einer Atmosphäre einen Dampfdruck von weniger als 1000 Pa aufweist.

3. Haarpflegezusammensetzung nach Anspruch 1 oder Anspruch 2, wobei die Siliconverbindung Polysiloxan ist, umfassend Dimethicon, Dimethiconol, Amodimethicon, Dimethiconol-Derivate oder Mischungen davon.

4. Haarpflegezusammensetzung nach Anspruch 3, wobei die Siliconverbindung Dimethiconol-Derivate darstellt, einschließlich Dimethiconol/ Silsesquioxan-Copolymer, Dimethiconol-Arginin, Dimethiconol-Bienenwachs, Dimethiconol-Behenat, Dimethiconol-Cystein, Dimethiconol-Meadowfoamat, Dimethiconol-Methionin, Dimethiconol-Panthenol, Amodimethicon, Dimethiconol-Stearat, Trimethylsiloxysilicat/ Dimethiconol-Crosspolymer, Acrylate/ Dimethiconolacrylat-Copolymer oder Mischungen davon, vorzugsweise Dimethiconol/ Silsesquioxan-Copolymer.

5. Haarpflegezusammensetzung nach einem der vorhergehenden Ansprüche, wobei die Zusammensetzung die Silikonverbindung in einer Menge von 0,1 bis 10%, bezogen auf das Gewicht der Zusammensetzung, vorzugsweise von 0,5 bis 5%, umfasst.

6. Haarpflegezusammensetzung nach einem der vorhergehenden Ansprüche, wobei das lineare oder verzweigte Alkan Octan, Isooctan, Decan, Isodecan, Dodecan, Isododecan, Tetradecaon, Isotetradecan, Hexadecan, Isohexydecan, Octodecan, Isooctadecan, Eicosan, Isoeicosan, Docosan, Isodocosan oder Mischungen davon umfasst.

7. Haarpflegezusammensetzung nach Anspruch 6, wobei das lineare oder verzweigte Alkan Isooctan, Isodecan, Isododecan, Isotetradecan, Isohexadecan, Isooctadecan, Isoeicosan, Isodocosan oder Mischungen davon, vorzugsweise Isohexadecan, umfasst.

8. Haarpflegezusammensetzung nach einem der vorhergehenden Ansprüche, wobei die Zusammensetzung das lineare oder verzweigte Alkan in einer Menge von 0,01 bis 5%, bezogen auf das Gewicht der Zusammensetzung, vorzugsweise von 0,05 bis 3%, umfasst.

9. Haarpflegezusammensetzung nach einem der vorhergehenden Ansprüche, wobei das Antischuppenmittel Piroctone-Olamin ist.

10. Haarpflegezusammensetzung nach einem der vorhergehenden Ansprüche, wobei die Zusammensetzung das Antischuppenmittel in einer Menge von 0,01 bis 10%, bezogen auf das Gewicht der Zusammensetzung, vorzugsweise von 0,01 bis 5%, umfasst.

11. Haarpflegezusammensetzung nach einem der vorhergehenden Ansprüche, wobei die Cyclomethicon-Verbindung Octamethylcyclotetrasiloxan, Decamethylcyclopentasiloxan, Dodecamethylcyclohexasiloxan oder Mischungen davon umfasst, wobei die Cyclomethicon-Verbindung vorzugsweise Decamethylcyclopentasiloxan ist.

12. Haarpflegezusammensetzung nach einem der Ansprüche 1 bis 10, wobei die Cyclomethicon-Verbindung in der Zusammensetzung fehlt.

13. Haarpflegezusammensetzung nach einem der vorhergehenden Ansprüche, wobei das kationische Tensid Stearamidopropyldimethylamin, Behentrimoniumchlorid, Cetyltrimethylammoniumchlorid oder Mischungen davon umfasst.

14. Haarpflegezusammensetzung nach einem der vorhergehenden Ansprüche, wobei die Zusammensetzung zusätzlich einen Fettalkohol umfasst.

15. Haarpflegezusammensetzung nach Anspruch 14, wobei der Fettalkohol 8 bis 22 Kohlenstoffatome umfasst.

**Revendications**

1. Composition pour le soin des cheveux comprenant :

(a) un tensioactif cationique ;
(b) un composé de silicone ;
(c) un alcane linéaire ou ramifié ayant de 8 à 22 atomes de carbone ; et
(d) un agent antipelliculaire choisi parmi la piroctone olamine, le sulfure de sélénium, des agents anti-fongiques à base d'azote et des mélanges de ceux-ci ;
dans laquelle la composition est substantiellement exempte d'un composé de cyclométhicone ;
dans laquelle ledit composé de cyclométhicone est présent dans une quantité inférieure à 1,5 % sur la base de la masse totale de la composition pour le soin des cheveux.

2. Composition pour le soin des cheveux selon la revendication 1, dans laquelle le composé de silicone est un silicone non-volatile qui présente une pression de vapeur inférieure à 1 000 Pa à 25°C et une pression atmosphérique.

3. Composition pour le soin des cheveux selon la revendication 1 ou revendication 2, dans laquelle le composé de silicone est un polysiloxane comprenant la diméthicone, le diméthiconol, l'amodiméthicone, des dérivés de diméthiconol ou des mélanges de ceux-ci.

4. Composition pour le soin des cheveux selon la revendication 3, dans laquelle le composé de silicone est des dérivés de diméthiconol incluant un copolymère de diméthiconol/silsesquioxane, la diméthiconol arginine, la diméthiconol cire d'abeille, le béhénate de diméthiconol, la diméthiconol cystéine, le diméthiconol meadowfoamate, la diméthiconol méthionine, le diméthiconol panthénol, l'amodiméthicone, le diméthiconol stéarate, un polymère croisé de triméthylsiloxysilicate/ diméthiconol, un copolymère d'acrylates/acrylate de diméthiconol ou des mélanges de ceux-ci, de préférence un copolymère de diméthiconol/silsesquioxane.

5. Composition pour le soin des cheveux selon l'une quelconque des revendications précédentes, dans laquelle la composition comprend le composé de silicone dans une quantité de 0,1 à 10 % en masse de la composition, de préférence de 0,5 à 5 %.

6. Composition pour le soin des cheveux selon l'une quelconque des revendications précédentes, dans laquelle l'alcane linéaire ou ramifié comprend l'octane, isooctane, décane, isodécane, dodécane, isododécane, tétradécane, isotétradécane, hexadécane, isohexadécane, octodécane, isooctadécane, éicosane, isoéicosane, docosane, isodocosane ou des mélanges de ceux-ci.

7. Composition pour le soin des cheveux selon la revendication 6, dans laquelle l'alcane linéaire ou ramifié comprend l'isooctane, isodécane, isododécane, isotétradécane, isohexadécane, isooctadécane, isoéicosane, isodocosane ou des mélanges de ceux-ci, de préférence isohexadécane.

8. Composition pour le soin des cheveux selon l'une quelconque des revendications précédentes, dans laquelle la composition comprend l'alcane linéaire ou ramifié dans une quantité de 0,01 à 5 % en masse de la composition, de préférence de 0,05 à 3 %.

9. Composition pour le soin des cheveux selon l'une quelconque des revendications précédentes, dans laquelle l'agent antipelliculaire est la piroctone olamine.

10. Composition pour le soin des cheveux selon l'une quelconque des revendications précédentes, dans laquelle la composition comprend l'agent antipelliculaire dans une quantité de 0,01 à 10 % en masse de la composition, de préférence de 0,01 à 5 %.

11. Composition pour le soin des cheveux selon l'une quelconque des revendications précédentes, dans laquelle le composé de cyclométhicone comprend l'octaméthylcyclotétrasiloxane, décaméthylcyclopentasiloxane, dodécaméthylcyclohexasiloxane ou des mélanges de ceux-ci, le composé de cyclométhicone est de préférence le décaméthylcyclopentasiloxane.

12. Composition pour le soin des cheveux selon l'une quelconque des revendications 1 à 10, dans laquelle le composé de cyclométhicone est absent dans la composition.

13. Composition pour le soin des cheveux selon l'une quelconque des revendications précédentes, dans laquelle le tensioactif cationique comprend la stéaramidopropyl diméthylamine, le chlorure de béhéntrimonium, chlorure de cétyltriméthylammonium ou des mélanges de ceux-ci.

**14.** Composition pour le soin des cheveux selon l'une quelconque des revendications précédentes, dans laquelle la composition comprend de plus un alcool gras.

**15.** Composition pour le soin des cheveux selon la revendication 14, dans laquelle l'alcool gras comprend de 8 à 22 atomes de carbone.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2017133874 A

- WO 9631188 A